# EUROPEAN PATENT APPLICATION

(11) **EP 4 183 794 A1**
(43) Date of publication of application: **24.05.2023**
(21) Application number: 21843299.5
(22) Date of filing: 16.07.2021
(51) Int. Cl.: C07K 7/08, A61K 38/17, A61P 9/14, A61P 19/02, C07K 7/00, C07K 14/47

(54) **PEPTIDE, PEPTIDE SALT, PHARMACEUTICAL COMPOSITION AND BIOLOGICAL TISSUE CALCIFICATION INHIBITOR**

(30) Priority: 17.07.2020 JP 2020122547
(71) Applicant: Hiroshima University, Higashihiroshima-shi Hiroshima 739-8511 (JP)
(72) Inventor: YOSHIKO, Yuji, Higashihiroshima-shi, Hiroshima 739-8511 (JP); MINAMIZAKI, Tomoko, Higashihiroshima-shi, Hiroshima 739-8511 (JP); TOSHISHIGE, Masaaki, Higashihiroshima-shi, Hiroshima 739-8511 (JP); KOHNO, Shohei, Higashihiroshima-shi, Hiroshima 739-8511 (JP)
(74) Representative: Epping - Hermann - Fischer
(86) International application number: PCT/JP2021/026751
(87) International publication number: WO 2022/014697

(57) **Abstract**

A peptide or salt thereof includes an amino acid sequence set forth in SEQ ID NO:1 containing an amino acid sequence in which at least one of (a) the first aspartate residue and the second aspartate residue from the N-terminus, or (b) the 17th glycine residue and the 18th aspartate residue from the N-terminus is deleted. A peptide or salt thereof includes an amino acid sequence set forth in SEQ ID NO:5 containing an amino acid sequence in which the sixth aspartate residue from the N-terminus is substituted with another amino acid residue. The peptides or salts thereof include three specific serine residues of which at least two serine residues are phosphorylated, and have an action of inhibiting biotissue calcification.

## Description

### Technical Field

The present disclosure relates to a peptide, a salt of the peptide, a pharmaceutical composition, and a biotissue calcification inhibitor.

### Background Art

Examples of calcification of biotissues include calcification of hard tissues and ectopic calcification. The calcification of hard tissues refers to calcification of bones and teeth. The ectopic calcification is calcification occurring in the soft tissues of the whole body due to renal dysfunction, arteriosclerosis, metabolic disorder, aging, heredity, and/or the like. Examples of the soft tissues in which the calcification occurs include the vessel, the ligament, the tendon, the skeletal muscle, the alveolar wall, the kidney, the gastric mucosa, the periarticular tissue, the cartilage, and the skin. More specific examples of the ectopic calcification include vascular calcification caused by chronic kidney disease or arteriosclerosis, as well as generalized arterial calcification of infancy (GACI) and fibrodysplasia ossificans progressive (FOP). The ectopic calcification causes pain, movement disorder, ulceration, and the like, and the vascular calcification also affects vital prognosis.

A small integrin-binding ligand N-linked glycoprotein (SIBLING) is a protein included in the hard tissue matrix. Cleavage of the SIBLING results in a peptide fragment including an acidic serine and aspartate-rich motif (ASARM) (hereinafter referred to as "ASARM peptide").

An ASARM peptide resulting from matrix extracellular phosphoglycoprotein (MEPE) which is one of SIBLINGs has high binding affinity with hydroxyapatite, and binds to an apatite crystal *in vitro* to inhibit matrix calcification caused by osteoblasts (Non Patent Literature 1 and Non Patent Literature 2).

Patent Literature 1 discloses a hard tissue calcification inhibitor and an ectopic calcification inhibitor using an active site related to inhibition of calcification of an ASARM peptide derived from MEPE.

### Citation List

### Patent Literature

Patent Literature 1: Japanese Patent Application Publication No. 2013-14566

### Non Patent Literature

Non Patent Literature 1: P. S. N. Rowe and 11 others, "MEPE has the properties of an osteoblastic phosphatonin and minhibin", Bone, 2004, 34(2), 303-319
Non Patent Literature 2: William N Addison and four others, "MEPE-ASARM Peptides Control Extracellular Matrix Mineralization by Binding to Hydroxyapatite: An Inhibition Regulated by PHEX Cleavage of ASARM", J. Bone Miner Res., 2008, 23(10), 1638-1649

### Summary of Invention

### Technical Problem

An ASARM peptide derived from human MEPE includes three (Asp-Xs) sites to which amino acid residues (X) other than an aspartate residue is adjacent at the C-terminal side of the aspartate residue (Asp). The Asp-Xs tend to result in formation of aspartimide, causing intramolecular dehydration. The ASARM peptide is immediately metabolized *in vivo.* Improvement in stability is desired even for reducing a dosage in consideration of application to medicines.

The present disclosure was made under such circumstances with an objective to provide a peptide with further enhanced stability, a salt of the peptide, a pharmaceutical composition, and a biotissue calcification inhibitor.

### Solution to Problem

In order to solve the problem described above, the present inventors modified an ASARM peptide on the basis of stability and biotissue calcification inhibitory activity as indices. As a result, a peptide having high stability and high biotissue calcification inhibitory activity was found, and the present disclosure was thus accomplished.

A peptide or salt thereof according to a first aspect of the present disclosure includes:
an amino acid sequence set forth in SEQ ID NO: 1 containing
   an amino acid sequence in which at least one of (a) a first aspartate residue and a second aspartate residue from an N-terminus, or (b) a 17th glycine residue and an 18th aspartate residue from the N-terminus is deleted, wherein
   at least two serine residues are phosphorylated among three serine residues corresponding to 11th, 13th and 15th serine residues from the N-terminus, wherein
the peptide or salt thereof has an action of inhibiting biotissue calcification.

A peptide or salt thereof according to a second aspect of the present disclosure includes:
an amino acid sequence set forth in SEQ ID NO:5 containing
   an amino acid sequence in which a sixth aspartate residue from an N-terminus is substituted with another amino acid residue, wherein
   at least two serine residues are phosphorylated among three serine residues corresponding to ninth, 11th, and 13th serine residues from the N-terminus, wherein
the peptide or salt thereof has an action of inhibiting biotissue calcification.

It is also acceptable that the peptide or salt thereof according to the second aspect of the present disclosure includes an amino acid sequence set forth in SEQ ID NO:7.

It is also acceptable that the peptide or salt thereof according to the first and second aspects of the present disclosure is cyclic.

It is also acceptable that the at least three serine residues are phosphorylated.

A pharmaceutical composition according to a third aspect of the present disclosure includes the peptide or salt thereof according to the first or second aspect of the present disclosure.

A biotissue calcification inhibitor according to a fourth aspect of the present disclosure includes the peptide or salt thereof according to the first or second aspect of the present disclosure.

### Advantageous Effects of Invention

In accordance with the present disclosure, the stability of a peptide, a salt of the peptide, a pharmaceutical composition, and a biotissue calcification inhibitor can be further enhanced.

### Brief Description of Drawings

FIG. 1 is a view illustrating the amino acid sequence of human MEPE, the position of an ASARM peptide, and sites cleaved by cathepsin B in the human MEPE;
FIG. 2 is a view illustrating part of the amino acid sequence of the ASARM peptide and sites to which amino acid residues other than an aspartate residue are adjacent at the C-terminal side of the aspartate residue;
FIG. 3 is a view illustrating the *in vitro* calcification inhibitory activities of peptides according to Examples;
FIG. 4 is a view illustrating the mouse thoracic aortae of which the calcified tissues were stained;
FIG. 5 is a view illustrating the *in vivo* calcification inhibitory activity of peptides according to Examples;
FIG. 6 is a view illustrating the residual rates of peptides according to Examples in phosphate buffered saline (PBS);
FIG. 7 is a view illustrating the residual rates of peptides according to Examples in mouse serum;
FIG. 8 is a view illustrating the calcification inhibitory activities of peptides according to Examples in a GACI model;
FIGS. 9A and 9B are views illustrating the calcification inhibitory activity of a peptide according to Examples in an FOP model, FIG. 9A is the view illustrating µCT images of the leg regions of a mouse as a negative control and a mouse to which the peptide according to Examples was administered, and FIG. 9B is the view illustrating a µCT image of the leg region of an untreated mouse 10 days after administration of Ad-Cre; and
FIGS. 10A to 10D are views illustrating the results of evaluation of the influence of a peptide according to Examples on osteogenesis, and illustrating a bone volume, a bone mineral content, a bone mineral density, and a bone strength, respectively.

### Description of Embodiments

Embodiments according to the present disclosure are described with reference to the drawings. The present disclosure is not limited to the following embodiments and drawings. Descriptions of peptides in the embodiments are also applied to salts thereof.

### Embodiment 1

A peptide according to the present embodiment is a peptide designed based on a human ASARM peptide. The peptide has an action of inhibiting biotissue calcification. The biotissue calcification in such a case is, for example, at least one of hard tissue calcification or ectopic calcification. "Ectopic calcification" is particularly "vascular calcification". Examples of the vascular calcification include: vascular calcification associated with renal disease or dialysis; and vascular calcification caused by arteriosclerosis. "Hard tissue" means a hard biotissue, and examples thereof include bone tissues forming the skeletons of the cranium and the trunk, the alveolar bone and cement included in the paradentium, and the dentine (of which examples also include cells included therein). Preferably, the hard tissue is a bone tissue.

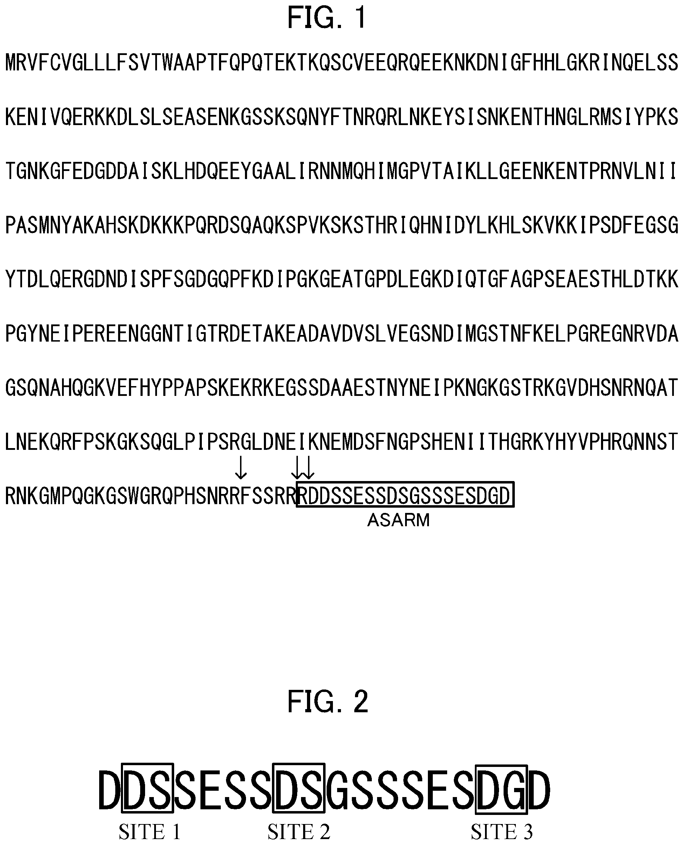

FIG. 1 is a view illustrating sites cleaved by cathepsin B in the amino acid sequence of human MEPE set forth in SEQ ID NO:2, and the position of an ASARM peptide resulting from the cleavage by the cathepsin B. The arrows in FIG. 1 indicates the sites cleaved by cathepsin B.

An amino acid sequence (SEQ ID NO: 1) included in the amino acid sequence of the ASARM peptide includes a site 1 ("DS") consisting of the second and third amino acid residues from the N-terminus, a site 2 ("DS") consisting of the eighth and ninth amino acid residues from the N-terminus, and a site 3 ("DG") consisting of the 16th and 17th amino acid residues from the N-terminus, as sites to which amino acid residues other than an aspartate residue are adjacent, in the C-terminal side of an aspartate residue, as illustrated in FIG. 2.

The peptide according to the present embodiment includes an amino acid sequence in which at least one of the site 1 or the site 3 is modified in the amino acid sequence set forth in SEQ ID NO: 1. More specifically, the amino acid sequence of the peptide according to the present embodiment, in which the site 1 is modified, is an amino acid sequence in which the first aspartate residue and the second aspartate residue from the N-terminus are deleted in the amino acid sequence set forth in SEQ ID NO: 1. The amino acid sequence is an amino acid sequence set forth in SEQ ID NO:3.

The amino acid sequence of the peptide according to the present embodiment, in which the site 3 is modified, is an amino acid sequence in which the 17th glycine residue and the 18th aspartate residue from the N-terminus are deleted in the amino acid sequence set forth in SEQ ID NO: 1. The amino acid sequence is set forth in SEQ ID NO:4.

The amino acid sequence of the peptide according to the present embodiment may be an amino acid sequence in which the first aspartate residue, the second aspartate residue, the 17th glycine residue, and the 18th aspartate residue from the N-terminus are deleted in the amino acid sequence set forth in SEQ ID NO: 1. The amino acid sequence is set forth in SEQ ID NO:5.

The peptide according to the present embodiment may be a peptide including an amino acid sequence in which the site 2 is modified. Specifically, the amino acid sequence in which the site 2 is modified is an amino acid sequence in which the sixth aspartate residue from the N-terminus is substituted with another amino acid residue in the amino acid sequence set forth in SEQ ID NO:5. The other amino acids replaced with aspartic acid are not particularly limited as long as the peptide according to the present embodiment maintains a biotissue calcification inhibitory action. The other amino acids are preferably asparagine and glutamine residues having polarities similarly with an aspartate residue, and more preferably a glutamate residue which is acidic. When such another amino acid residue is glutamic acid, the peptide according to the present embodiment includes, for example, an amino acid sequence set forth in SEQ ID NO:6 or 7. The amino acid sequence of the peptide according to the present embodiment preferably includes an amino acid sequence set forth in SEQ ID NO:7. Such anther amino acid replaced with aspartic acid may be a corresponding amino acid in an ASARM peptide derived from the MEPE of a species except a human, for example, serine.

One or two of Asp-Xs that tend to result in formation of aspartimide in the peptide set forth in SEQ ID NO:1 are deleted by modifying one or two of the sites 1 and 3 as described above. Any Asp-X that tends to result in formation of aspartimide in the ASARM peptide is deleted in the peptide including the amino acid sequence in which the site 2 is modified. As a result, the peptide according to the present embodiment results in inhibition of formation of aspartimide in comparison with the ASARM peptide. In particular, in the case of the peptide including the amino acid sequence set forth in SEQ ID NO:7, all the three Asp-Xs are deleted, and therefore, the formation of aspartimide is further inhibited in comparison with the ASARM peptide.

When the peptide according to the present embodiment includes an amino acid sequence in which the sixth aspartate residue from the N-terminus is substituted with another amino acid residue in the amino acid sequence set forth in SEQ ID NO:5, the number of amino acid residues included in the peptide is not particularly limited if the number is 14 or more as long as a biotissue calcification inhibitory action is maintained, and the number is, for example, 14 to 25, 14 to 23, 14 to 21, and 14 to 19.

A salt of the peptide according to the present embodiment is not particularly limited as long as being a pharmaceutically acceptable salt, and may be either an acid salt or a basic salt. Examples of the acid salt include: inorganic acid salts such as a hydrochloride, a sulfate, a nitrate, and a phosphate; organic acid salts such as an acetate, a trifluoroacetic acid (TFA) salt, a citrate, a maleate, a malate, an oxalate, a lactate, a succinate, a fumarate, and a propionate; and the like. Examples of the basic salt include: alkali metal salts such as a sodium salt and a potassium salt; an alkali earth metal salt such as a calcium salt and a magnesium salt; and the like.

The peptide according to the present embodiment may be either a straight-chain peptide or a cyclic peptide. The term "cyclic" means a closed ring structure formed in the molecule by binding two amino acids, which are two or more amino acid residues apart from each other in the straight-chain peptide, to each other directly, or through a linker or the like. When the peptide according to the present embodiment is a cyclic peptide, the peptide may be allowed to be cyclic in an optional aspect. For example, a peptide allowed to be cyclic is obtained by linking the carboxyl and amino termini of the peptide according to the present embodiment to each other through a direct amide bond or a peptide linker. The peptide may also be allowed to be cyclic by linking functional groups in the side chains of amino acids included in the peptide according to the present embodiment or the peptide linker. An aspect of the cyclic peptide is not limited to bonding of the amino acids of the N-terminus and C-terminus of the straight-chain peptide to each other, and the cyclic peptide may be formed by binding the amino acid of a terminal and an amino acid other than the amino acid of a terminal to each other, or binding amino acids other than the amino acids of the terminals to each other.

The peptide according to the present embodiment is preferably a cyclic peptide that is allowed to be cyclic by linking the N-terminus and C-terminus thereof to each other through intramolecular bonding between functional groups. Examples of the cyclic peptide include a peptide allowed to be cyclic by an intramolecular bond through the functional group of an amino acid added to the N-terminus or C-terminus of the peptide according to the present embodiment. The intramolecular bond is not particularly limited as long as being a covalent bond, and examples thereof include a disulfide bond, a thioether bond, a peptide bond, an ether bond, an ester bond, an alkyl bond, an alkenyl bond, a phosphonate ether bond, an azo bond, a C-S-C bond, a C-N-C bond, a C= N-C bond, an amide bond, a lactam crosslink, a carbamoyl bond, a urea bond, a thiourea bond, an amine bond, and a thioamide bond. When two amino acids are bound to each other in the main chain of amino acids, a closed ring structure is formed by a peptide bond. The covalent bond between the two amino acids may be formed by binding between the side chains of the two amino acids or between the side and main chains of the two amino acids, or the like. The intramolecular bond is preferably a thioether bond.

As the cyclic peptide according to the present embodiment, each of cyclic peptides in various aspects can be adopted as long as the amino acid sequence of an acyclic peptide in which at least one of the sites 1 to 3 described above is modified is maintained. For example, through a peptide bond between the amino group of the N-terminus and the carboxyl group of the C-terminus of each of the amino acid sequences set forth in SEQ ID NOS:3 to 7, the N-terminus and the C-terminus may be linked to each other.

The cyclic peptide may be formed by chemistry modifying at least one of the N-terminus or the C-terminus and then linking the N-terminus and the C-terminus to each other. For example, the amino group of the N-terminus may be acetylated, a cysteine residue may be added to the C-terminus, and the N-terminus and the C-terminus may be linked to each other through a thioether bond between the acetyl group of the N-terminus and the thiol group of the cysteine residue of the C-terminus. The amino group of the N-terminus may be chloroacetylated, a cysteine residue may be added to the C-terminus, and the N-terminus and the C-terminus may be linked through a thioether bond between the chloroacetyl group of the N-terminus and the thiol group of the cysteine residue of the C-terminus.

For example, a cysteine residue may be added to each of the N-terminus and the C-terminus, and a disulfide bond may be formed between the thiol group of the cysteine residue of the N-terminus and the thiol group of the cysteine residue of the C-terminus, to link the N-terminus and the C-terminus to each other.

When the amino acid sequence of the peptide according to the present embodiment includes an amino acid sequence set forth in SEQ ID NO:1 containing an amino acid sequence in which at least one of (a) the first aspartate residue and the second aspartate residue from the N-terminus, or (b) the 17th glycine residue and the 18th aspartate residue from the N-terminus is deleted, and containing at least two serine residues are phosphorylated among three serine residues corresponding to 11th, 13th and 15th serine residues from the N-terminus. In the peptide according to the present embodiment, all of the three serine residues are preferably phosphorylated. In the peptide according to the present embodiment, at least one of the serine residues other than the three serine residues may be phosphorylated, or all the serine residues may be phosphorylated.

When the peptide according to the present embodiment includes an amino acid sequence set forth in SEQ ID NO:5 containing an amino acid sequence in which the sixth aspartate residue from the N-terminus is substituted with another amino acid residue, and containing at least two serine residues are phosphorylated among three serine residues corresponding to ninth, 11th, and 13th serine residues from the N-terminus. In the peptide according to the present embodiment, all of the three serine residues are preferably phosphorylated. The peptides in which the ninth, 11th, and 13th serine residues from the N-termini are phosphorylated in the amino acid sequences set forth in SEQ ID NOS:5 and 7 are set forth in SEQ ID NOS:8 and 9, respectively.

The peptide according to the present embodiment can be synthesized by a known method. For example, the peptide may be synthesized by a chemical polypeptide synthesis method, or may be synthesized by a genetic engineering method using *E. coli* and/or the like. Examples of the polypeptide synthesis method may include a liquid phase method such as a fragment condensation method, or a solid phase method such as an Fmoc method. The liquid phase method is a method in which a reaction is performed in a solution state, a product is isolated and purified from the reaction mixture, and the obtained product is used as an intermediate product in a subsequent peptide elongation reaction. In contrast, the solid phase method is a method in which amino acids are bound to a solid phase support insoluble in a reaction solvent, and the amino acids are subjected in turn to a condensation reaction to elongate a peptide. The above-described peptide can also be synthesized by condensing a partial peptide or amino acids included in the peptide with the residual portion, and desorbing a protecting group when a product includes the protecting group. The above-described peptide synthesis method can also be performed using a commercially available automatic synthesizer.

The phosphorylation of a serine residue can be performed by a known method such as a prephosphorylation method in which a protected phosphorylated amino acid is condensed, and the protecting group of phosphoric acid is removed in the final stage of synthesis.

The obtained peptide may be purified or analyzed by, for example, recrystallization, ion exchange chromatography, high performance liquid chromatography, reversed-phase chromatography, affinity chromatography, an Edman degradation method, and/or the like.

A cyclic peptide is obtained by cyclizing an acyclic peptide obtained by the method described above. A known method may be adopted as a method of cyclizing an acyclic peptide. For example, in a case in which the N-terminus of the peptide is modified with an acetyl halide group, examples of the method include a method in which the halogenated acetyl group of the residue and the thiol group of a cysteine residue added to the C-terminus are allowed to react with each other in a basic buffer to form a thioether bond to thereby cyclize the acyclic peptide.

Examples of a method of cyclizing a peptide through a peptide bond include a dehydration condensation reaction using dicyclohexylcarbodiimide and/or the like, a symmetric acid anhydride method, and an active ester method. Examples of a method of cyclizing a peptide through a disulfide bond include an air oxidation method and an oxidation method using potassium ferricyanide.

In the cyclization reaction described above, an oligomer in which acyclic peptides are linked through an intermolecular bond, as well as the cyclic peptide, may be formed depending on a reaction condition. Accordingly, the peptide subjected to a cyclization reaction is preferably purified by reversed phase high performance liquid chromatography and/or the like in order to obtain only the cyclic peptide.

At least three sites in which formation of aspartimide is prone to occur exist in the sequence of the ASARM peptide, so that it has been difficult to synthesize the ASARM peptide, and the ASARM peptide has had poor stability. In contrast, the peptide according to the present embodiment can be improved in stability by removing the sites in which the formation of aspartimide is prone to occur. In accordance with the peptide according to the present embodiment, the inhibition of the formation of aspartimide facilitates synthesis of the peptide to allow a reduction in the production cost of the peptide to be expected.

Further, the peptide according to the present embodiment has a high biotissue calcification inhibitory activity, as described in the following examples. The ASARM peptide is immediately metabolized *in vivo,* and therefore, continuous administration of a high dose of the ASARM peptide is considered to be required for obtaining sufficient drug efficacy. However, the peptide according to the present embodiment has such a high activity, and therefore allows sufficient drug efficacy to be obtained while reducing the dose of the peptide according to the present embodiment.

In another embodiment, with regard to the peptide described above, a peptide is provided in which another amino acid is substituted so that the peptide has an action of inhibiting biotissue calcification. For example, an amino acid other than the sixth glutamate residue from the N-terminus in the amino acid sequence set forth in SEQ ID NO:7 may be substituted with an amino acid in an ASARM peptide derived from the MEPE of a species except a human. The position of the amino acid to be substituted can be determined by aligning the above-described peptide and the ASARM peptide for reference by a known method. For example, the 14th aspartic acid from the N-terminus in the amino acid sequence set forth in SEQ ID NO:7 may be substituted with another amino acid residue. The other amino acid with which the aspartic acid is substituted is not particularly limited as long as the peptide according to the present embodiment maintains the biotissue calcification inhibitory action. Examples of the other amino acid with which the aspartic acid is substituted include histidine, serine, and asparagine.

The substitution with the amino acid described above may be conservative amino acid substitution. "Conservative amino acid substitution" refers to substitution of an amino acid residue with another amino acid residue including a similar side chain. In the conservative amino acid substitution, for example, a lysine residue including a basic side chain is substituted with an arginine residue including another basic side chain. For example, amino acid residues including similar side chains are classified into: amino acid residues including basic side chains such as lysine, arginine, and histidine; amino acid residues including acidic side chains such as aspartic acid and glutamic acid; amino acid residues including uncharged polar side chains such as glycine, asparagine, glutamine, serine, threonine, tyrosine, and cysteine; amino acid residues including nonpolar side chains such as alanine, valine, leucin, isoleucine, proline, phenylalanine, methionine, and tryptophan; amino acid residues including β-branched chains such as valine, leucin, and isoleucine; and amino acid residues including aromatic side chains such as tyrosine, phenylalanine, tryptophan, and histidine.

### Embodiment 2

A pharmaceutical composition according to the present embodiment includes the peptide according to Embodiment 1 as described above. The pharmaceutical composition includes the above-described peptide as an active component. The pharmaceutical composition is preferably a biotissue calcification inhibitor. Hereinafter, the biotissue calcification inhibitor is described as the pharmaceutical composition.

The biotissue calcification inhibitor according to the present embodiment is, for example, a liquid, a tablet, a granule, a subtle granule, a powder, a tablet, a capsule, or the like. The content of the peptide according to Embodiment 1 as an active component in the biotissue calcification inhibitor is adjusted as appropriate.

Items for use of the biotissue calcification inhibitor according to the present embodiment are cells, biotissues, organisms, animals, and the like. In addition, for a method of use, and the like, an appropriate use amount and means of use may be used in consideration of the potency of the calcification inhibitor, a substance, an animal, an organism, or the like as a target, and the degree of the calcification state of the target.

Examples of diseases for which the biotissue calcification inhibitor according to the present embodiment is used include GACI, FOP, ossification of the posterior longitudinal ligament (OPLL), ossification of the yellow ligament, arthrosis deformans, and vascular calcification. The biotissue calcification inhibitor as a pharmaceutical product is administered to humans and animals. The animals are preferably mammals, of which more specific examples include dogs, cats, cattle, pigs, horses, sheep, and deer.

The route of administration of the biotissue calcification inhibitor according to the present embodiment to a human or the like is not particularly limited. The biotissue calcification inhibitor is preferably used as an injectable agent or an orally-administered agent. In such a case, the biotissue calcification inhibitor may include, for example, the above-described peptide and a pharmaceutically acceptable carrier. Such pharmaceutically acceptable carriers are various organic carrier substances or inorganic carrier substances used as formulation materials. The pharmaceutically acceptable carriers are, for example, excipients, lubricants, binders, and disintegrants in solid preparations, or solvents, solubilizing agents, suspending agents, isotonizing agents, buffer agents, and soothing agents in liquid preparations. An additive such as an antiseptic agent, an antioxidant, a coloring agent, or a sweetener may also be optionally blended.

The biotissue calcification inhibitor according to the present embodiment is produced by a known method, and includes 0.000001 to 99.9% by weight, 0.00001 to 99.8% by weight, 0.0001 to 99.7% by weight, 0.001 to 99.6% by weight, 0.01 to 99.5% by weight, 0.1 to 99% by weight, 0.5 to 60% by weight, 1 to 50% by weight, or 1 to 20% by weight of the above-described peptide as an active component.

The dose of the biotissue calcification inhibitor according to the present embodiment is determined as appropriate depending on, for example, the age, body weight, and symptom of a human or animal to which the biotissue calcification inhibitor is administered. The biotissue calcification inhibitor is administered so that the amount of the above-described peptide is an effective amount. The effective amount is the amount of the peptide, necessary for obtaining a desired result, and an amount necessary for resulting in delay of progression, inhibition, prevention, reversal, or cure of a condition to be subjected to therapy or treatment, for example, calcification of a biotissue.

The dose of the biotissue calcification inhibitor according to the present embodiment is typically 0.01 mg/kg to 1000 mg/kg, preferably 0.1 mg/kg to 200 mg/kg, and more preferably 0.2 mg/kg to 20 mg/kg, and the biotissue calcification inhibitor can be administered one or more times per day. The biotissue calcification inhibitor may also be administered in various dosing frequencies such as every day, every other day, once a week, every other week, and once a month. Preferably, the dosing frequency can be easily determined by a doctor and/or the like. A dose outside the ranges described above can also be optionally used.

The biotissue calcification inhibitor according to the present embodiment may also be used as means for research on, for example, inhibition of the progression of a medical condition of arthrosis deformans or vascular calcification.

The biotissue calcification inhibitor according to the present embodiment is considered to be more resistant to decomposing *in vivo* than an ASARM peptide because of including the peptide improved in stability. As a result, the biotissue calcification inhibitor according to the present embodiment can be applied to an administration method corresponding to a medical condition. In addition, the biotissue calcification inhibitor according to the present embodiment has a high biotissue calcification inhibitory activity, and therefore, the dose of the biotissue calcification inhibitor can be reduced.

In order to improve the blood stability of the peptide according to the present embodiment, the peptide may be modified with a sugar chain or polyethylene glycol (PEG). In the case of the cyclic peptide, a cyclization method in which the stability is improved is preferably adopted. The stability may also be improved by substituting, with another amino acid, the above-described amino acid that can be substituted. A known drug delivery system (DDS) such as preparation of liposome may also be used to efficiently transport the peptide to a target organ or tissue.

In another embodiment, a reagent including the peptide according to Embodiment 1 as described above is provided. In another embodiment, the biotissue calcification inhibitor described above can also be used as an oral composition for inhibiting calcification of a biotissue. Specific examples of the oral composition include supplements, food compositions, foods or drinks, functional foods, and food additives.

The form of such a supplement is not particularly limited, and may be an optional form such as a tablet, a powdered agent, a granule, a capsule, a sugar-coated tablet, a film agent, a lozenge, a chewable agent, a solution, an emulsion, or a suspension. The supplement may include an optional component that is normally used in a supplement.

"Functional food" means a food or beverage that is ingested for the purpose of preserving health, and examples thereof include: specified health foods and functional nutritional foods, which are functional health foods; health foods; and nutritional supplementary foods. The functional food is preferably a specified health food or a functional nutritional food, which is a functional health food. In the case of commercialization as the functional food, various additives used in foods, specifically, a coloring agent, a preservative, a thickening stabilizer, an antioxidant, a bleaching agent, an antimicrobial/antifungal agent, an acidulant, a sweetener, a seasoning, an emulsifier, a nutrient, an agent for production, a flavoring agent, and/or the like may be added. Foods and beverages that are functional foods are not particularly limited. Examples of the form of the functional food include beverages, confectionery, grain-processed products, fish-paste products, dairy products, and seasonings.

The biotissue calcification inhibitor described above may be added as a food additive to a food. In such a case, the food additive may be allowed to be a paste, a gelatinous agent, a powdered agent, a liquid, a suspension, an emulsion, a granule, or the like so as to be easily added to the food.

In another embodiment, a method of inhibiting calcification of a biotissue by administering the peptide according to Embodiment 1 as described above to a patient is provided. Another embodiment is use of the peptide according to Embodiment 1 as described above for inhibiting calcification of a biotissue. In another embodiment, the peptide according to Embodiment 1 as described above for use as a biotissue calcification inhibitor is provided. Another embodiment is use of the peptide according to Embodiment 1 as described for producing a biotissue calcification inhibitor.

### Examples

The present disclosure is further specifically described with reference to the following Examples. However, the present disclosure is not limited to Examples.

### (Synthesis of Peptide)

An ASARM peptide (Comparative Example 1) including an amino acid sequence set forth in SEQ ID NO:10, and a peptide (Comparative Example 2, SEQ ID NO:11) in which the 12th, 14th, and 16th serines from the N-terminus of the amino acid sequence of Comparative Example 1 were phosphorylated were synthesized. A peptide (Example 1, SEQ ID NO: 12) which included an amino acid sequence set forth in SEQ ID NO:4, and in which the 11th, 13th, and 15th serine residues from the N-terminus were phosphorylated, a peptide (Example 2, SEQ ID NO:8) which included an amino acid sequence set forth in SEQ ID NO:5, and in which the ninth, 11th, and 13th serine residues from the N-terminus were phosphorylated, a peptide (Example 3, SEQ ID NO:9) which included an amino acid sequence set forth in SEQ ID NO:7, and in which the ninth, 11th, and 13th serine residues from the N-terminus were phosphorylated, and a peptide (Example 4, SEQ ID NO: 13) which included the amino acid sequence set forth in SEQ ID NO:7, and in which the 11th and 13th serines from the N-terminus were phosphorylated were synthesized.

A cyclic peptide (Example 5) in which the peptide of Example 3 was cyclized was synthesized. In the synthesis of Example 5, the amino group of the N-terminus of the peptide including the amino acid sequence set forth in SEQ ID NO:9 was chloroacetylated, a cysteine residue was added to the C-terminal amino group, and the carboxyl group of the cysteine residue was amidated. Subsequently, the chloroacetyl group of the N-terminus and the thiol group of the cysteine residue of the C-terminus were linked to each other through a thioether bond.

A peptide including phosphorylated serine was synthesized by an immobilization method using Fmoc-Ser (PO(OBzl)OH)-OH as a building block.

The peptides were purified by high efficiency liquid chromatography (HPLC) using an ODS column and 0.1% TFA/acetonitrile, and had purities of 90 to 95%. The peptides were prepared with a TFA salt (of which part was an ammonium salt).

### (Examination of In Vitro Calcification Inhibitory Activity)

A mouse osteoblast strain MC3T3-E1 was cultured in a bone differentiation medium (α MEM (Eagle's minimum essential medium, alpha modification) + 50 µg/mL ascorbic acid, 10% fetal calf serum) to form osteoid-like nodules. In order to induce calcification, 3 mM β-glycerophosphoric acid (hereinafter, "βGP") was added together with a test substance to the medium. The strain was cultured for 24 hours after the addition of the test substance, and cells were washed with PBS, immobilized with 10% neutral buffered formalin, and then subjected to von Kossa staining. The matrix calcification was evaluated by measurement of calcium spots stained with von Kossa in the extracellular matrices of alkaline phosphatase (ALP)-stained positive cells, which were osteoblastic markers, with a stereoscopic microscope.

### (Results)

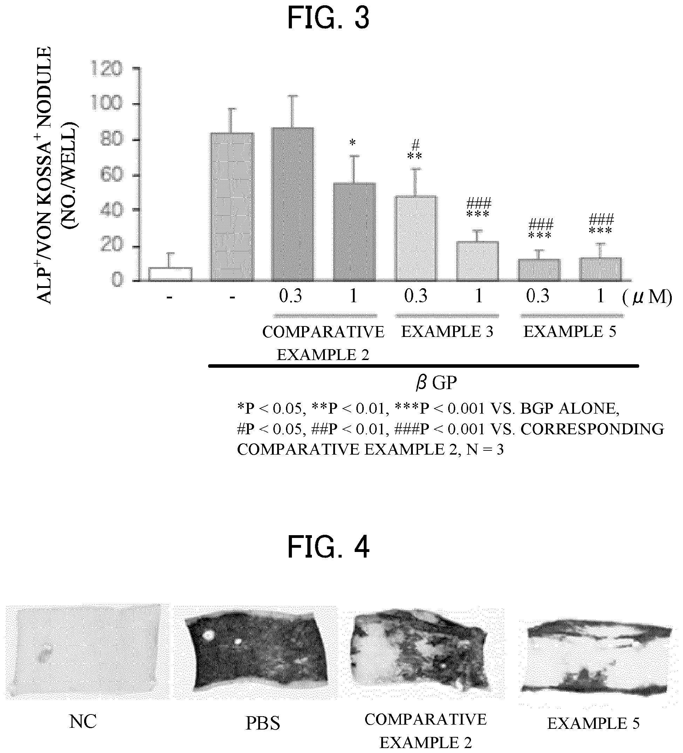

FIG. 3 illustrates the calcification inhibitory activities in Comparative Example 2, and Examples 3 and 5. Table 1 sets forth the relative calcification inhibitory activities in Comparative Example 1, and Examples 1 to 5 in a case in which the calcification inhibitory activity in Comparative Example 2 was set at 100. In Table 1, the serine residues under which double underlines are put are phosphorylated serine residues.

**[Table 1]**

| | Amino acid sequence | Calcification inhibitory activity |
|---|---|---|
| Comparative Example 1 | RDDSSESSDSGSSSESDGD (SEQ ID NO: 10) | 0 |
| Comparative Example 2 | RDDSSESSDSGSSSESDGD (SEQ ID NO: 11) | 100 |
| Example 1 | DDSSESSDSGSSSESD (SEQ ID NO:12) | 100 |
| Example 2 | SSESSDSGSSSESD (SEQ ID NO:8) | 100 |
| Example 3 | SSESSESGSSSESD (SEQ ID NO:9) | 200< |
| Example 4 | SSESSESGSSSESD (SEQ ID NO: 13) | 110< |
| Example 5 | Ac-SSESSESGSSSESD(C)-NH₂ | 500-1000< |

### (Examination of In Vivo Calcification Inhibitory Activity)

The thoracic aorta was harvested from a C57BL/6 mouse (10-week-old male), and then cut into ring-shaped portions (2 to 3 mm), which were cultured in 10% FCS DMEM (Dulbecco's Moodified Eagle's Medium, high glucose). From the next day, the peptides were added, and the resultants were loaded together with phosphoric acid having a high concentration (sodium phosphate, final concentration of 3 mM), and cultured for 10 days. The tissues immobilized with PFA were stained with alizarin red, and the vessel walls were incised and opened, and observed with a stereoscopic microscope. Moreover, the contents of calcium in liquid extracts obtained by decalcifying the vessels, cultured in a similar manner, with 10% formic acid for 24 hours were measured using a calcium quantification kit (Calcium C-test Wako, manufactured by Wako Pure Chemical Industries, Ltd.).

### (Results)

FIG. 4 illustrates the tissues of the thoracic aorta, imaged with the stereoscopic microscope. The calcified regions were stained purplish red. NC indicates a negative control. The substantially whole region was calcified in PBS which was a solvent for the peptide, whereas calcification was inhibited in Comparative Example 2 and Example 5. The calcification inhibitory effect in Example 5 was superior to that in Comparative Example 2.

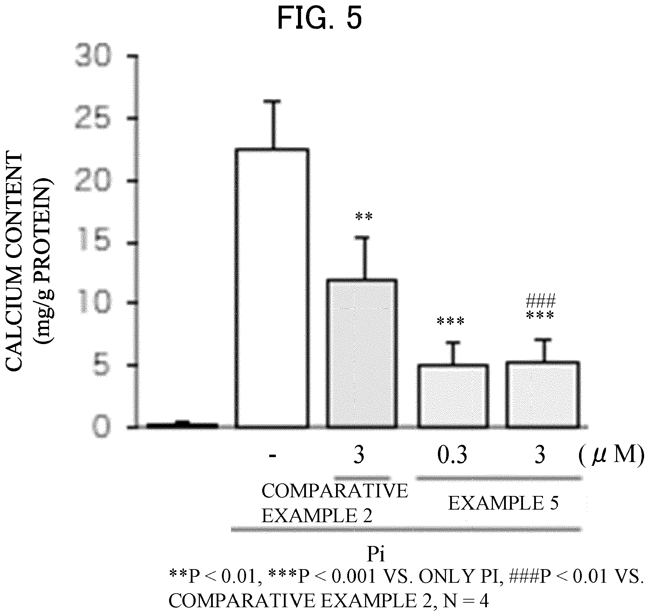

FIG. 5 illustrates a calcium content per protein. Pi indicates load with phosphoric acid. The calcium contents in Example 5 were less than that in Comparative Example 2. Calcification in Example 5 is shown to be more greatly inhibited than that in Comparative Example 2.

### (Evaluation of Stability)

The amount of 1/20 to 1/25 of Comparative Example 2 or Example 5 (each 100 mM) was added to PBS or C57BL/6 mouse serum, and incubated at 37°C for 7 days or 14 days. In measurement using the serum, 4M perchloric acid (PCA) of which the amount was equal to the amount of a sample was added, and the resultant was diluted to 2-fold with MQ water and left to stand on ice for 5 minutes. The supernatant obtained by centrifugation for 2 minutes was subjected to HPLC (ODS column and 0.1% TFA/ acetonitrile). The residual rates of the test substances were calculated in a case in which the peak area of a sample adjusted before use was set at 100.

### (Results)

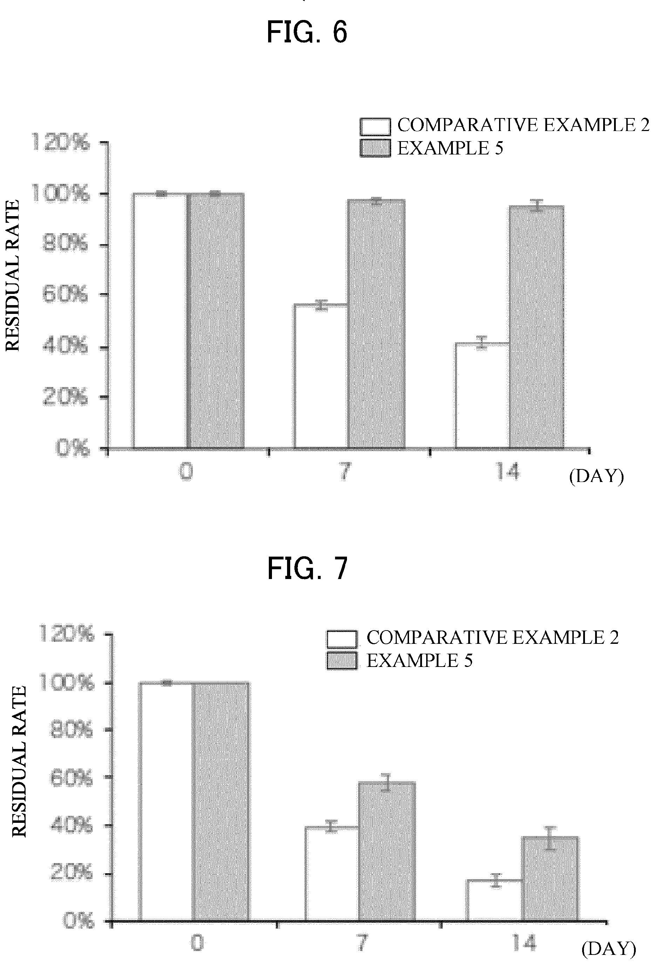

FIG. 6 illustrates the residual rates in PBS. The residual rate was decreased over time in Comparative Example 2, whereas the residual rate was not decreased in Example 5. FIG. 7 illustrates the residual rates in the mouse serum. A decrease in the residual rate in the serum in Example 5 was inhibited in comparison with Comparative Example 2.

### (Evaluation of Drug Efficacy in GACI Model)

The biallelic mutation of an Enpp1 gene is observed at 75% of GACI. Thus, the drug efficacy of the peptide was examined using Enpp1 mutated mice. Fertilized eggs from the Enpp1 mutated mice (C57BL/6J-Enpp1^{asj/asj}) were transplanted into ICR mice, and bred with a high phosphorus diet (AIN-93G-based, 0.4% Ca, 0.85% P, 0.04% Mg) immediately after deliveries. Male Enpp1^{asj} mice were weaned from the mothers 4 weeks after the births, and fed with a high phosphorus diet during an experimental period. The Enpp1^{asj} mice bred under such conditions exhibited the calcification of the aorta little by little from 6 weeks after the births, and 80% or more of the mice exhibited from 8 to 10 weeks after the births. A catheter was indwelled in the right cervical vein 6 to 7 weeks after the birth, and Example 5 was continuously administered for 2 weeks by an osmotic pump (manufactured by Altez). The thoracic (ventral) aorta was harvested, then immobilized with 4% para-formaldehyde PBS, washed, and then stained with alizarin red 0.5% KOH. The tissue was washed, and then preserved in 70% EtOH. For the obtained tissue, the calcification of the alizarin red-positive region of the thoracic aorta was scored (maximum value of 3) under the stereoscopic microscope.

### (Results)

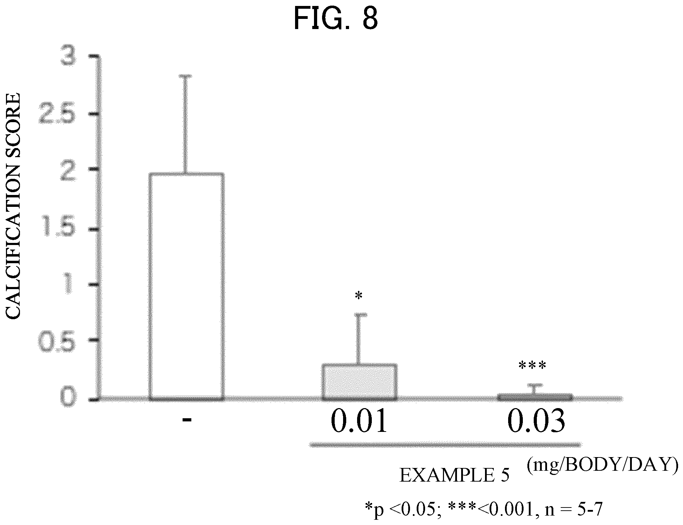

FIG. 8 illustrates calcification scores. By intravenous administration of Example 5, vascular calcification in the GACI model mice was inhibited.

### (Evaluation of Drug Efficacy in FOP Model)

Cre recombinase recombinant adenovirus (Ad-Cre, manufactured by Vector Biolabs) was intramuscularly administered (2 × 10⁷ pfu) to the crural muscles (triceps muscles) of 7-day-old caALK2Q207D mice (Paul B Yu and the 14 others, "BMP type I receptor inhibition reduces heterotopic ossification" Nat Med, 2008, 14(12), 1363-1369). Next day, Example 5 was intraperitoneally administered (0.03 mg/mouse/day). On day 6, the leg region was immobilized with 4% para-formaldehyde PBS, and µCT images were acquired.

### (Results)

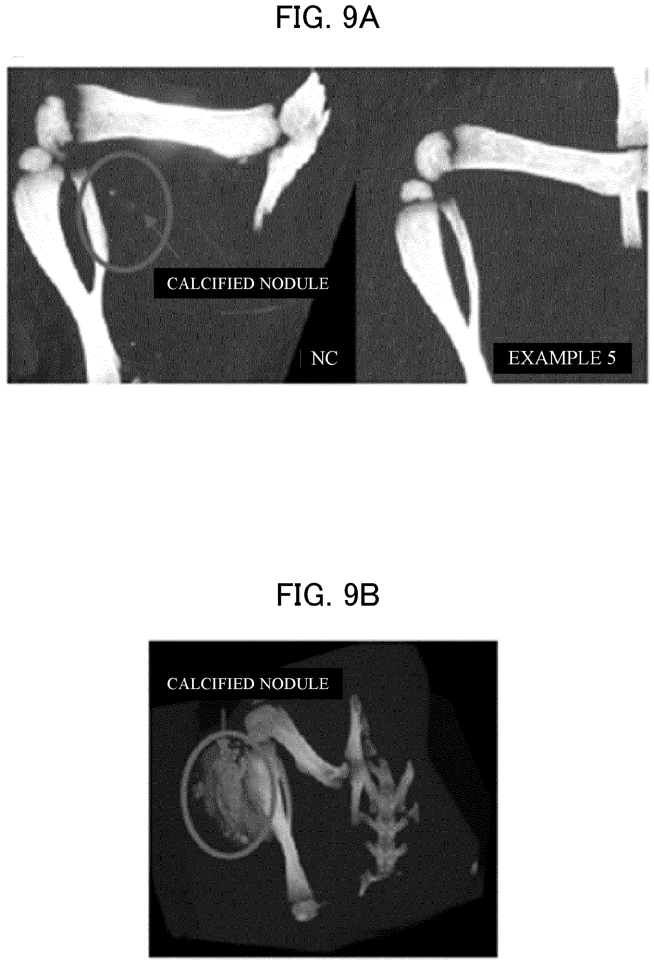

As illustrated in FIG. 9A, a calcified nodule was observed in the negative control, whereas no calcification was confirmed in a mouse receiving Example 5. FIG. 9B illustrates a µCT image of an untreated mouse 10 days after the administration of Ad-Cre.

### (Evaluation of Safety)

A phosphorylated ASARM peptide is an endogenous calcification inhibiting factor, actually inhibits matrix calcification caused by an osteoblast *in vitro* as examined above, and may therefore inhibit osteogenesis. Thus, toxicity, particularly an influence on osteogenesis was examined using Comparative Example 2, as described below.

Catheters were indwelled in the jugular veins of 6-week-old ddY mice, and Comparative Example 2 was continuously administered at a high dose (1 mg/mouse/day) or low dose (0.1 mg/mouse/day) for 4 weeks using an osmotic pump (manufactured by Alzert). The low dose is 10 or more times as much as an effective drug efficacy concentration. Evaluation items were set to a body weight, the amount of intake of a food and drink, blood biochemistry (including calcium and phosphoric acid), the weights of principal organs, and histopathology. Moreover, bone structures were evaluated by µCT, and bone strengths were evaluated in a three-point bending test.

### (Results)

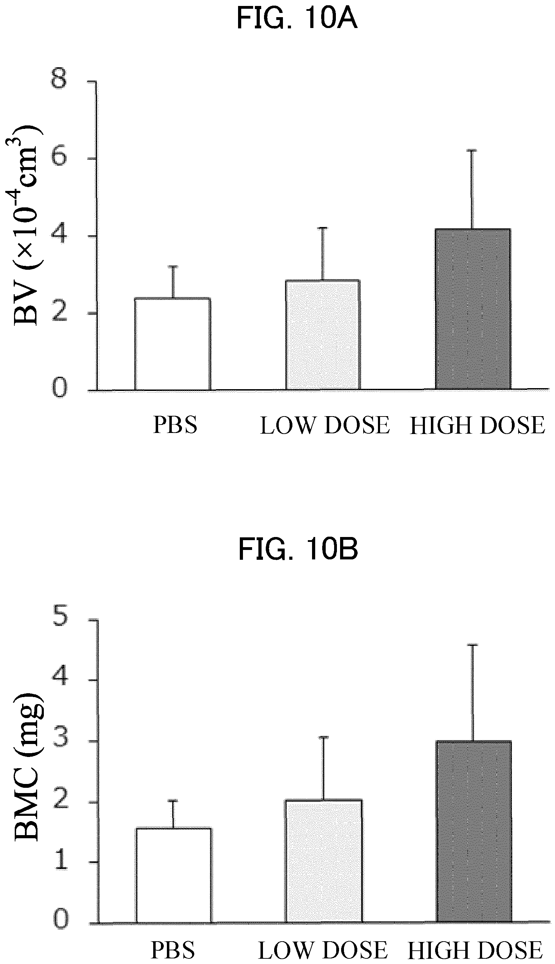

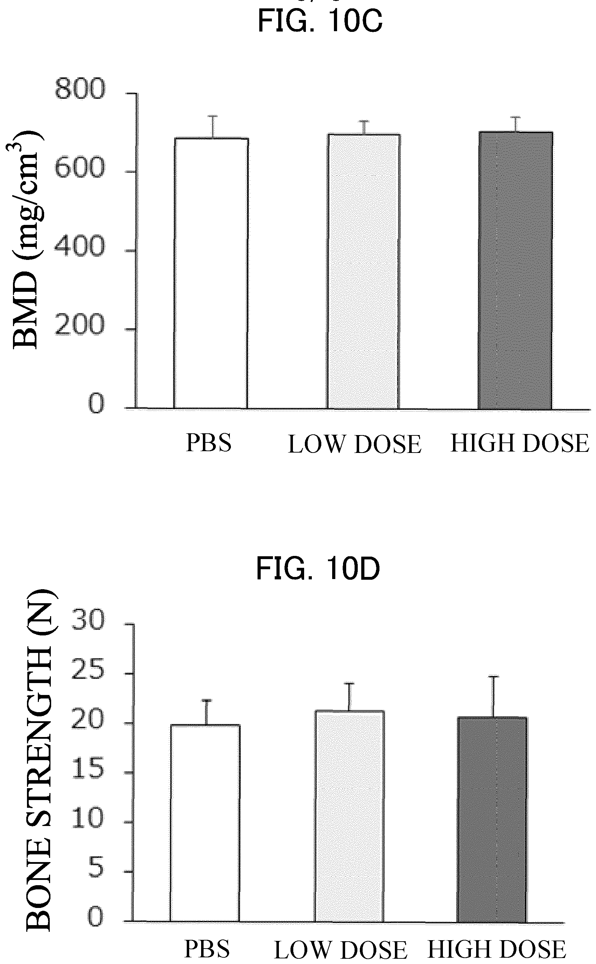

Any abnormal findings were not observed in the body weight, the amount of intake of a food and drink, the blood biochemistry, the weights of principal organs, and histopathology. FIGS. 10A, 10B, 10C, and 10D illustrate a bone volume (BV), a bone mineral content (BMC), a bone mineral density (BMD), and a bone strength (maximum load), respectively. There were no significant differences between PBS and all the examination items at both the high and low doses.

The safety of Comparative Example 2 was confirmed by the evaluation described above. Therefore, Examples 1 to 5 considered to result in calcification inhibitory action in the similar mechanism of action are considered to also have high safety.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2020-122547, filed on July 17, 2020, the entire disclosure of which is incorporated by reference herein.

### Industrial Applicability

The present disclosure is suitable for a medicine that inhibits calcification of a biotissue, such as hard tissue calcification or ectopic calcification.

## Claims

1. A peptide or salt thereof, comprising:
an amino acid sequence set forth in SEQ ID NO: 1 containing
an amino acid sequence in which at least one of (a) a first aspartate residue and a second aspartate residue from an N-terminus, or (b) a 17th glycine residue and an 18th aspartate residue from the N-terminus is deleted, wherein
at least two serine residues are phosphorylated among three serine residues corresponding to 11th, 13th and 15th serine residues from the N-terminus, wherein
the peptide or salt thereof has an action of inhibiting biotissue calcification.

2. A peptide or salt thereof, comprising:
an amino acid sequence set forth in SEQ ID NO:5 containing
an amino acid sequence in which a sixth aspartate residue from an N-terminus is substituted with another amino acid residue, wherein
at least two serine residues are phosphorylated among three serine residues corresponding to ninth, 11th, and 13th serine residues from the N-terminus, wherein
the peptide or salt thereof has an action of inhibiting biotissue calcification.

3. The peptide or salt thereof according to claim 2, comprising:
an amino acid sequence set forth in SEQ ID NO:7.

4. The peptide or salt thereof according to any one of claims 1 to 3, wherein the peptide or salt thereof is cyclic.

5. The peptide or salt thereof according to any one of claims 1 to 4, wherein the at least three serine residues are phosphorylated.

6. A pharmaceutical composition, comprising:
the peptide or salt thereof according to any one of claims 1 to 5.

7. A biotissue calcification inhibitor, comprising:
the peptide or salt thereof according to any one of claims 1 to 5.
